# EUROPÄISCHE PATENTSCHRIFT

(11) **EP 0 489 689 B1**
(45) Veröffentlichungstag und Bekanntmachung des Hinweises auf die Patenterteilung: **01.03.1995**
(21) Anmeldenummer: 91810925.7
(22) Anmeldetag: 27.11.1991
(51) Int. Cl.: C07C 50/36, C07C 50/24, G03C 1/73, C09K 9/02, C09B 3/58

(54) **Photochrome Naphthacenchinone, Verfahren zu deren Herstellung und deren Verwendung**
Photochromic naphthacenequinones, process for their production and their use
Naphtacènequinones photochromiques, procédé pour leur préparation et leur utilisation

(30) Priorität: 05.12.1990 CH 3837/90
(43) Veröffentlichungstag der Anmeldung: 10.06.1992
(73) Patentinhaber: CIBA-GEIGY AG, 4002 Basel (CH)
(72) Erfinder: Fischer, Walter, Dr., CH-4153 Reinach (CH); Finter, Jürgen, Dr., W-7800 Freiburg (DE); Spahni, Heinz, CH-4402 Frenkendorf (CH)

(56) Entgegenhaltungen:
- EP-A- 0 438 376
- DE-A- 2 337 855
- CHEMICAL ABSTRACTS, vol. 94, no. 7, Columbus, Ohio, US; abstract no. 47012M, Y.E GERASIMENKO ET AL.: 'photochromism of peri-arylhydroxy-p-quinones. Synthesis of some derivatives of phenoxynaphthacenequinones' Seite 548 ;

## Beschreibung

Die Erfindung betrifft in 5,12- bzw. 6,11-Stellung mit Aryloxygruppen und in den 2-, 3-, 8- und/oder 9-Stellungen mit mindestens einer Phenoxygruppe oder mindestens einem F-Cl- oder Br-Atom substituierte Naphthacen-6,11- bzw. Naphthacen-5,12-dione, entsprechende 5,12- bzw. 6,11-Dichlornaphthacendione, ein Verfahren zu deren Herstellung und deren Verwendung als photochrome Systeme zur Kontrastbildung, Lichtabsorption oder zur Aufzeichnung von Informationen.

Yu. E. Gerasimenko et al. beschreiben im Zhurnal Organicheskoi Khimii, Vol. 7, No. 11, S. 2413-2415 (1971) 6-Phenoxy-naphthacen-5,12-dion als reversible photochrome Verbindung, die bei Bestrahlung das orange 5-Phenoxynaphthacen-6,12-dion (Anachinon) bildet. Yu. E. Gerasimenko et al. beschreiben im Zhurnal Organicheskoi Khimii, Vol. 16, No. 9, S. 1938-1945 (1980) 6,11-Diphenoxy-naphthacen-5,12-dion, dessen Photoisomerisation zur Synthese von 6-Aminoderivaten des 12-Phenoxy-naphthacen-5,11-dions verwendet wird.

Ein Gegenstand der Erfindung sind Verbindungen der Formel I oder Mischungen solcher Verbindungen, 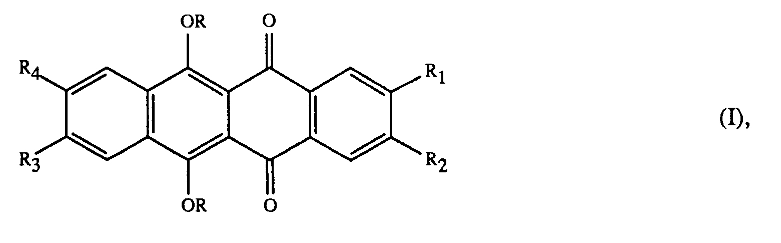
worin
R unsubstituiertes oder mit C₁-C₁₂-Alkyl, C₁-C₁₂-Alkoxy, C₁-C₁₂-Alkylthio, Phenyl, Benzyl, -CN, -CF₃, Halogen oder -COOR₅ substituiertes C₆-C₁₄-Aryl bedeutet und R₅ für H, C₁-C₁₈-Alkyl, Cyclohexyl, Cyclopentyl, Phenyl, C₁-C₁₂-Alkylphenyl, Benzyl oder C₁-C₁₂-Alkylbenzyl steht, und
mindestens einer der Reste R₁ bis R₄ -F, -Cl oder -Br darstellt oder unabhängig die Gruppe RO- bedeutet, und die anderen Reste von R₁ bis R₄ für -H, -F, -Cl oder -Br stehen.

R in Formel I ist bevorzugt unsubstituiertes oder substituiertes C₆-C₁₀-Aryl, zum Beispiel Phenyl, 1- oder 2-Naphthyl. Bevorzugt stellt R unsubstituiertes oder substituiertes Phenyl dar.

Die Gruppe R kann mit einem oder mehreren, bevorzugt 1 bis 3 Resten substituiert sein. Wenn R mit Alkyl, Alkoxy oder Alkylthio substituiert ist, können diese linear oder verzweigt sein und bevorzugt 1 bis 6, besonders 1 bis 4 C-Atome enthalten. Beispiele sind Methyl, Ethyl, die Isomeren von Propyl, Butyl, Pentyl, Hexyl, Heptyl, Octyl, Nonyl, Decyl, und die entsprechenden Alkoxy- und Alkylthioreste. Bevorzugt sind Methyl, Ethyl, n- und i-Propyl, n-, i- und t-Butyl, Methoxy, Ethoxy, Methylthio und Ethylthio.

Wenn R mit Halogen substituiert ist, handelt es sich bevorzugt um -Br, -Cl und -F.

R₅ in der Bedeutung von Alkyl kann linear oder verzweigt sein. Weitere Beispiele zu den zuvorgenannten Alkylresten sind die Isomeren von Tridecyl, Tetradecyl, Pentadecyl, Hexadecyl, Heptadecyl und Octadecyl. Bevorzugt enthält R₅ als Alkyl 1 bis 12, besonders 1 bis 6 C-Atome. R₅ als Alkylphenyl ist bevorzugt C₁-C₆-, besonders C₁-C₄-Alkylphenyl, z. B. Dodecylphenyl, Octylphenyl, Hexylphenyl, n-, i- oder t-Butylphenyl, n- oder i-Propylphenyl, Ethylphenyl, Methylphenyl. R₅ als Alkylbenzyl ist bevorzugt C₁-C₆-, besonders C₁-C₄-Alkylbenzyl, z.B. Dodecylbenzyl, Octylbenzyl, Hexylbenzyl, n-, i- oder t-Butylphenyl, n- oder i-Propylbenzyl, Ethylbenzyl, Methylbenzyl. R₅ ist bevorzugt H oder C₁-C₁₈-Alkyl, besonders C₁-C₁₂-Alkyl.

In einer bevorzugten Ausführungsform ist R in Formel I unsubstituiert oder mit C₁-C₄-Alkyl, C₁-C₄-Alkoxy, C₁-C₄-Alkylthio, -F, -Cl, -Br oder -COOR₅ substituiert, wobei R₅ -H oder C₁-C₁₈-Alkyl bedeutet.

In einer ganz besonders bevorzugten Ausführungsform ist R in Formel I unsubstituiert oder mit -Cl, -Br oder -COO(C₁-C₆)-Alkyl substituiert.

Eine andere bevorzugte Ausführungsform sind solche Verbindungen der Formel I, worin in Formel I mindestens einer der Reste R₁ bis R₄ eine Gruppe RO- oder -F, -Cl oder -Br darstellt, und die anderen Reste von R₁ bis R₄ für -H stehen.

In einer weiteren bevorzugten Ausführungsform sind R₁ oder R₄, oder R₁ und R₃ oder R₄, oder R₁ und R₂, oder R₁ bis R₄ eine Gruppe RO-, -F, -Cl oder -Br, besonders bevorzugt eine Gruppe RO- oder -Cl.

Bevorzugt sind Verbindungen der Formel I, worin R unsubstiuiertes Phenyl darstellen.

Besonders bevorzugte Verbindungen der Formel I sind 6,11-Diphenyloxy-2-chlor-naphthacen-5,12-dion, 2,6,11-Triphenyloxy-naphthacen-5,12-dion, 6,11-Diphenyloxy-2,3,8,9-tetrachlor-naphthacen-5,12-dion und 2,3,6,8,9,11-Hexaphenyloxy-naphthacen-5,12-dion.

Ein weiterer Gegenstand der Erfindung ist ein Verfahren zur Herstellung von Verbindungen der Formel I, bei dem man 1 Mol einer Verbindung der Formel II 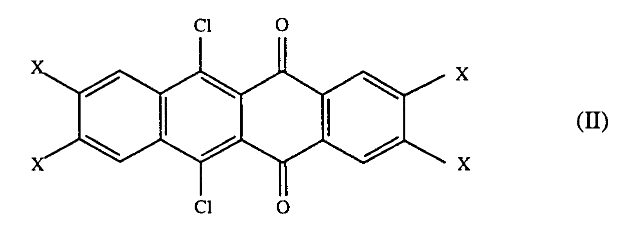
worin mindestens einer der Reste X für -F, -Cl oder -Br steht und die anderen Reste X für -H, -F, -Cl oder -Br stehen, in Gegenwart eines polaren aprotischen Lösungsmittels und bei erhöhter Temperatur mit mindestens 2 Mol einer Verbindung der Formel RO^{⊖}M^{⊕} umsetzt, worin R die zuvor angegebenen Bedeutungen hat und M für ein Alkalimetall oder tertiäres Ammonium mit 3 bis 18 C-Atomen steht.

Es wurde überraschend gefunden, dass die beiden Cl-Atome regioselektiv mit Phenoxygruppen substituiert werden können, auch wenn das Naphthacenringsystem weitere Halogenatome enthält.

Das erfindungsgemässe Verfahren wird bevorzugt bei Temperaturen von 50 bis 200°C, besonders 50 bis 150°C durchgeführt. Die Salze der Formel RO^{⊖}M^{⊕} können als solche eingesetzt werden oder durch Umsetzung eines entsprechenden Phenols mit einer Alkalimetallbase, Alkalimetallcarbonaten oder tertiären Aminen in situ im Reaktionsgemisch erzeugt werden. Die Salze können in äquimolaren Mengen oder im Ueberschuss, z.B. bis zu 40 Mol-% Ueberschuss eingesetzt werden, wenn eine Substitution von allen Halogenatomen erzielt werden soll.

Geeignete Lösungsmittel sind z.B. N-substituierte Carbonsäureamide und Lactame (z.B. Dimethylformamid oder N-Methylpyrrolidon), Sulfoxide und Sulfone (z.B. Dimethylsulfoxid, Tetramethylensulfon) oder Ether (z.B. n-Dipropylether, n-Dibutylether, Tetrahydrofuran oder Dioxan).

Die Isolierung und Reinigung der Verbindungen der Formel I erfolgt nach üblichen Methoden, z.B. durch Kristallisation und Umkristallisation, oder chromatographische Verfahren.

Die Verbindungen der Formel RO^{⊖}M^{⊕} sind bekannt oder in bekannter Weise durch die Umsetzung entsprechender Phenole mit Alkalimetallbasen , Alkalimetallcarbonaten oder tertiären Aminen erhältlich. Sie können im Reaktionsgemisch auch in situ erzeugt werden. Als Alkalimetallkationen kommen besonders Li^{⊕}, Na^{⊕} und K^{⊕} in Frage. Beispiele für tertiäres Ammonium sind Trimethyl-, Triethyl-, Tri-n-propyl- und Tri-n-butylammonium.

Die Verbindungen der Formel II sind nach folgendem Verfahren erhältlich:
Die Umsetzung der bekannten Verbindungen der Formel III 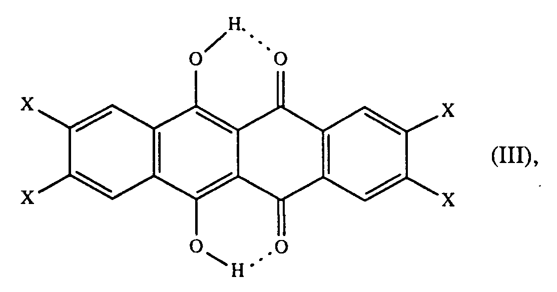
worin X die in Formel II angegebenen Bedeutungen hat, mit einem Chlorierungsmittel, zum Beispiel POCl₃, ergibt die Verbindungen der Formel IV, die bei unsymmetrischer Substitution als Gemisch von Stellungsisomeren der Formeln IV und IVa vorliegen:

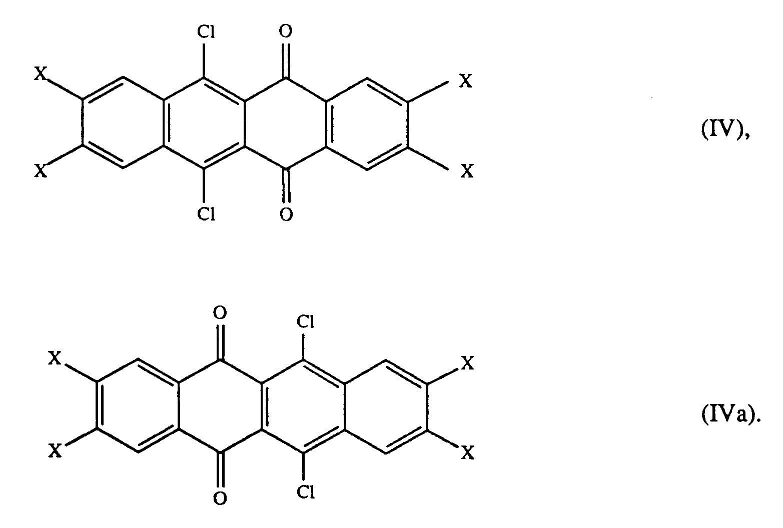

Solche Gemische können direkt zur Herstellung von Verbindungen der Formel II weiterverwendet werden oder vorher z.B. mit chromatographischen Methoden getrennt werden. Verbindungen der Formel III sind in an sich bekannter Weise zum Beispiel durch die Umsetzung von entsprechend halogenierten bzw. nichthalogenierten Phthalsäureanhydriden mit entsprechend halogenierten bzw. nicht halogenierten 1,4-Dihydroxynaphthalinen in Gegenwart von B₂O₃ bei erhöhten Temperaturen erhältlich.

Die Verbindungen der Formel I sind kristallin, thermisch stabil und hellgelb bis gelb-orange gefärbt. Sie sind in organischen Lösungsmitteln löslich. Sie sind wirksame Photoinitiatoren und Photosensibilisatoren für photopolymerisierbare Systeme, die ethylenisch ungesättigte Doppelbindungen enthalten. Ferner sind die Verbindungen der Formel I reversible Photochrome, die bei Bestrahlung eine deutliche Farbänderung von gelb bis gelb-orange nach orange bis rot aufweisen.

Bei Bestrahlung der erfindungsgemässen Verbindungen, gegebenenfalls in einem Substrat, mit Licht einer Wellenlänge von etwa 300 bis 450 nm wird eine ausgepragte Farbänderung nach orange bis rot beobachtet. Die Lichtabsorption ist im Vergleich zu 6,11-Diphenoxynaphthacen-5,12-dion zu höherer Wellenlänge verschoben. Die Farbänderung beruht auf der photochemischen Umwandlung der erfindungsgemässen Parachinone in die entsprechenden Anachinone der Formel V. Die Umwandlungsgeschwindigkeit ist überraschend hoch und kann je nach Menge, Dicke der Probe und Strahlungsintensität unter 3 Sekunden liegen.

Ein weiterer Gegenstand der Erfindung sind die Anachinone der Formel V 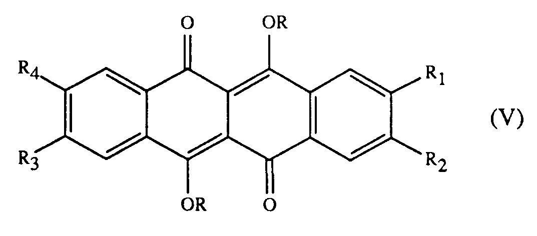
worin R, R₁, R₂, R₃ und R₄ die zuvor angegebenen Bedeutungen haben, einschliesslich der Bevorzugungen.

Die Verbindungen der Formel V können nach der Bestrahlung von Lösungen der Verbindungen der Formel I durch Entfernen des Lösungsmittels erhalten und gegebenenfalls nach üblichen Methoden gereinigt werden.

Die Farbänderung ist reversibel. Bei erneuter Bestrahlung mit Licht einer Wellenlänge von etwa 450 bis 550 nm erhält man wieder die ursprüngliche Farbe (Rückbildung der Parachinonstruktur). Besonders vorteilhaft ist, dass dieser Vorgang mehrfach wiederholt werden kann. Die Stabilität der photochemischen Hin- und Rückreaktion ist überraschend hoch und die Ermüdung selbst an Luft oder in Substraten entsprechend gering. So werden bei mehr als 200 Zyklen praktisch keine Veränderungen beobachtet. Als vorteilhaft ist auch anzusehen, dass die zur photochemischen Umwandlung benötigte Lichtabsorption im Bereich der Wellenlänge handelsüblicher Laser liegt.

Ein weiterer Gegenstand der Erfindung ist die Verwendung von Verbindungen der Formeln I oder V oder Gemischen davon als reversible photochrome Systeme zur Kontrastbildung oder Lichtabsorption.

Die Verbindungen der Formel I können als Photoinitiatoren und besonders Photosensibilisatoren in photopolymerisierbaren Systemen verwendet werden, wobei sie gleichzeitig als Farbindikatoren wirken. So ist es möglich, belichtete Produkte (z.B. Schutzschichten, Druckplatten, Offsetdruckplatten, gedruckte Schaltungen, Lötstopmasken) zu markieren und von unbelichteten Produkten zu unterscheiden, und ferner bei einer Produktekontrolle fehlerhaft belichtete Produkte vor oder nach der Entwicklung auszusondern.

Der erhebliche Vorteil bei der Verwendung als Farbindikatoren liegt in der Erhöhung der Sensibilisatorwirkung. Ueblicherweise als Farbumschlagsysteme eingesetzte Zusätze bewirken im allgemeinen eine Erniedrigung der Photosensibilität.

Die Verbindungen der Formeln I oder V können auch als solche, in Lösung oder in Polymeren eingearbeitet, als Photofarbindikatoren oder als Photoschaltelemente verwendet werden.

Die Verbindungen der Formel I können auch in organischen oder anorganischen Gläsern als photoschaltbare Farbfilter verwendet werden, z.B. in Gläsern für Sonnenbrillen, Kontaktlinsen, Fenster und Spiegel.

Ein weiterer Gegenstand der Erfindung ist eine strahlungsempfindliche Zusammensetzung, enthaltend
a) ein strahlungsempfindliches organisches Material, und
b) mindestens eine Verbindung der Formeln I oder V oder Mischungen davon.

Die Verbindungen der Formel I und V oder Mischungen davon können in einer Menge von 0,001 bis 20 Gew.-%, besonders 0,001 bis 10 Gew.-% und insbesondere 0,01 bis 5 Gew.-% enthalten sein, bezogen auf die Komponente a).

Strahlungsempfindliche und damit auch photostrukturierbare Materialien sind bekannt. Es kann sich um Positiv- oder Negativsysteme handeln. Solche Materialien sind z.B. von G. E. Green et al. in J. Macromol. Sci.; Revs. Macromol. und Chem., C21(2), 187-273 (1981 bis 1982) und von G.A. Delzenne in Adv. Photochem., 11, S. 1-103 (1979) beschrieben worden.

Vorzugsweise handelt es sich bei dem strahlungempfindlichen organischen Material um a1) eine nichtflüchtige monomere, oligomere oder polymere Substanz mit photopolymerisierbaren oder photodimerisierbaren ethylenisch ungesättigten Gruppen, a2) um ein kationisch härtbares System oder a3) um photovernetzbare Polyimide.

Photopolymerisierbare Substanzen sind z.B. Acryl- und besonders Methacrylsäureester von Polyolen, z.B. Ethylenglykol, Propandiol, Butandiol, Hexandiol, Di(hydroxymethyl)cyclohexan, Polyoxyalkylendiole wie z.B. Di-, Tri- oder Tetraethylenglykol, Di- oder Tri-1,2-propylenglykol, Trimethylolmethan, -ethan oder -propan und Pentaerythrit, die alleine, in Mischungen und in Abmischung mit Bindemitteln verwendet werden können.

Photodimerisierbare Substanzen sind z.B. Homo- und Copolymere, die Zimtsäuregruppen oder substituierte Maleinimidylverbindungen in Seitengruppen oder Chalkongruppen in der Polymerkette enthalten.

Bevorzugt sind solche Zusammensetzungen, worin Komponente a1) ein Homo- oder Copolymer von Acryl-, Methacryl- oder Maleinsäureestern ist, deren Estergruppen einen Rest der Formel 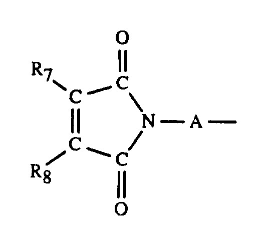
enthalten, worin A für unsubstituiertes oder mit Hydroxyl substituiertes lineares oder verzweigtes C₂-C₁₂-Alkylen, Cyclohexylen oder Phenylen steht, und R₇ und R₈ unabhängig voneinander Cl, Br, Phenyl oder C₁-C₄-Alkyl bedeuten, oder R₇ und R₈ zusammen Trimethylen, Tetramethylen oder 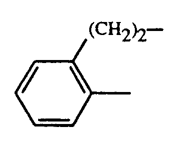
bedeuten. Solche Polymere sind z.B. in der US-A-4 193 927 beschrieben.

Die photopolymerisierbaren oder photodimerisierbaren Substanzen können weitere für die Verarbeitung oder Anwendung übliche Additive enthalten, sowie zusätzlich andere Photoinitiatoren oder Photosensibilisatoren.

Bei den kationisch härtbaren Systemen handelt es sich bevorzugt um Epoxidverbindungen mit mindestens 2 Epoxidgruppen im Molekül, denen ein Photoinitiator einverleibt ist. Geeignete Photoinitiatoren sind z.B. Cyclopentadienyl-Aren-Metallsalze, Cyclopentadienylmetallcarbonyl-salze und Oniumsalze, die z.B. in den zuvor erwähnten Publikationen beschrieben sind. Die härtbaren Systeme können für die Verarbeitung und Anwendung übliche Zusatzstoffe enthalten.

Photoempfindliche Polyimide sind z.B. in der DE-A-1 962 588, EP-A-0 132 221, EP-A- 0 134 752, EP-A-0 162 017, EP-A-0 181 37 und EP-A-0 182 745 beschrieben.

Die erfindungsgemässe Zusammensetzung wird nach bekannten Methoden als Schicht auf Substrate aufgebracht und entweder durch flächige Bestrahlung eine Schutzschicht oder durch Bestrahlung unter einer Photomaske oder durch ortsaufgelöste Bestrahlung mittels eines geführten Laserstrahls oder durch holographische Verfahren und nachfolgende Entwicklung ein Reliefbild erzeugt.

Ein weiterer Gegenstand der Erfindung ist eine Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche eine Verbindung der Formel I oder V oder Mischungen davon. Die Komponente b) ist bevorzugt in einer Menge von 0,001 bis 20 Gew.-%, besonders 0,001 bis 10 Gew.-% und insbesondere 0,01 bis 5 Gew.-% enthalten, bezogen auf Komponente a). Organische Lösungen können zur Beschichtung von anderen Substanzen verwendet werden, z.B. festen Substraten wie zum Beispiel anorganischen Gläsern, die dann als photoschaltbare Substrate verwendet werden können. Die Verbindungen der Formel I oder V können auch auf Substrate aufsublimiert werden. Die beschichteten Substrate können mit einer Schutzschicht aus z.B. transparenten Polymeren versehen werden. Feste Substrate können auch mit Zusammensetzungen beschichtet werden, die ein Polymer und mindstens eine Verbindung der Formeln I oder V enthalten. Geeignete Lösungsmittel sind z.B. Kohlenwasserstoffe, halogenierte Kohlenwasserstoffe, Ketone, Carbonsäureester und Lactone, N-alkylierte Säureamide und Lactame, Alkanole und Ether.

Geeignete Polymere sind z.B. Duroplaste, Thermoplaste und strukturell vernetzte Polymere. Die Polymeren sind bevorzugt transparent. Solche Polymere und organische Gläser sind dem Fachmann geläufig. Die Einarbeitung der erfindungsgemässen Verbindungen erfolgt nach üblichen Methoden, z.B. mit Lösungsverfahren und Entfernen des Lösungsmittels, Kalandrieren oder Extrusion. Die erfindungsgemässen Verbindungen können den Substraten auch vor, während oder nach deren Herstellung einverleibt werden.

Ein weiterer Gegenstand der Erfindung ist ein Verfahren zur Herstellung von gefärbten Materialien unter Einwirkung von Licht, dadurch gekennzeichnet, dass man dem Material eine Verbindung der Formel I oder V einverleibt und darauf das Material mit Licht bestrahlt.

Ferner ist ein Gegenstand der Erfindung die Verwendung von Verbindungen der Formel I als Photosensibilisatoren und Farbindikatoren oder photoschaltbare Farbfilter bei Lichteinwirkung.

Ein weiterer Gegenstand der Erfindung ist die Verwendung der Verbindung der Formel I oder V zur reversiblen optischen Speicherung von Information, wobei die Information in einer die Verbindung enthaltenden, speicheraktiven Schicht mit Licht, bevorzugt Laserlicht, eingeschrieben wird. Die eingeschriebene Information kann mit bevorzugt Laserlicht, wieder gelöscht werden, so dass ein zyklisches Einschreiben und Löschen möglich ist.

Zur Herstellung einer speicheraktiven Schicht kann die Verbindung I oder V nach zuvor beschriebenen Verfahren in einer transparenten Matrix gelöst und in dünner Schicht auf ein ebenes Substrat aufgebracht werden. Die Dicke der speicheraktiven Schicht beträgt im alllgemeinen ca 0,1-100 »m, bevorzugt 0,3-3 »m.

Das Einschreiben der Information kann durch gerasterte, holographische oder photographische Belichtung der speicheraktiven Schicht mit spektralem, bevorzugt kohärentem (Laser-)Licht im Wellenlängenbereich 440-550 nm, bevorzugt 460-530 nm erfolgen.

Das Auslesen kann mit reduzierter Strahlungsleistung bei der Einschreibwellenlänge über die örtlich geänderte Transmission, Reflexion, Brechung oder Fluoreszenz der speicheraktiven Schicht erfolgen.

Das Löschen kann durch punktförmige oder flächige Belichtung der speicheraktiven, die Verbindungen der Formeln I und /oder V enthaltenden Schicht im Wellenbereich 300-450 nm, bevorzugt 300-420 nm erfolgen.

Ein Vorteil der erfindungsgemässen Verwendung ist, dass die zum Einschreiben, Auslesen und Löschen erforderlichen Wellenlängen im Bereich handelsüblicher Laser liegen (z.B. Argonionenlaser: 488/514 nm bzw. 351/363 nm; Neodym-YAG-Laser: 532 nm bzw. 355 nm bei Frequenzverdopplung und Verdreifachung; XeF-Excimerlaser: 351 nm; HeCd-Laser: 325 und 442 nm).

Ein weiterer Vorteil ist der hohe erreichbare Kontrast der Absorption zwischen dem beschriebenen und dem gelöschten Zustand im Bereich 450-550 nm und die damit verbundene grosse Dynamik der speicheraktiven Schicht.

Ein anderer Vorteil ist, dass die Quantenausbeute beim Einschreiben relativ niedrig ist und damit die Gefahr eines Ueberschreibens beim Auslesen stark reduziert wird.

Vorteilhaft ist auch umgekehrt, dass die Quantenausbeute beim Löschvorgang relativ hoch ist und damit ein rasches grossflächiges Löschen ermöglicht wird.

Ein weiterer Vorteil ist, dass die Verbindung beim Auslesen fluoresziert und somit eine hochsensitive Detektion des Speicherzustands über die Fluoreszierung ermöglicht. Dass die zum Auslesen eingestrahlte Energie wesentlich über die Fluoreszierung und nicht thermisch dissipiert, wirkt zudem einer unerwünschten Erwärmung der speicheraktiven Schicht entgegen.

Ein weiterer Vorteil ist die hohe Photostabilität der Verbindung und die dadurch erreichbare hohe Zahl von Schreib-/Löschzyklen.

Schliesslich besteht als weiterer Vorteil die Möglichkeit einer zyklischen Datenauffrischung durch Zumischen eines geeigneten Quantums Licht der Löschwellenlänge während des Auslesens.

Die nachfolgenden Beispiele erläutern die Erfindung.

### A) Herstellung von Ausgangsverbindungen

Beispiel A1: 2,3,6,8,9,11-Hexachlor-naphthacen-5,12-dion.
30 g (70 mmol) 2,3,8,9-Tetrachlor-6,11-dihydroxy-naphthacen-5,12-dion, 60 ml POCl₃ und 500 ml o-Dichlorbenzol werden 90 Stunden unter Rückfluss gerührt. Darauf wird der Ueberschuss des POCl₃ zusammen mit dem o-Chlorbenzol abdestilliert, bis das Reaktionsvolumen noch etwa 300 ml beträgt. Nach dem Abkühlen wird der Niederschlag abfiltriert, mehrmals mit Wasser, wässriger Sodalösung gewaschen, getrocknet, mit Cyclohexan verrührt, abfiltriert und dann getrocknet. Ausbeute: 28,6 g (88 %),Schmelzpunkt (Smp.): >260 °C.

Beispiel A2: 2-Fluor- und 9-Fluor-6,11-dichlor-naphthacen-5,12-dion (Isomerengemisch).
3,0 g (9,73 mmol) 2- bzw. 9-Fluor-6,11-dihydroxy-naphthacen-5,12-dion (Gemisch der Stellungsisomeren), 4 ml POCl₃ und 30 ml o-Dichlorbenzol werden 10 Stunden am Rückfluss gerührt. Das Reaktionsgemisch wird auf Wasser gegossen und mit 2N wässriger NaOH neutralisiert. Der gebildete Niederschlag wird abfiltriert, mit Wasser gewaschen, getrocknet und in 400 ml heissem Toluol aufgenommen. Nach Zugabe von 20 g basischem Aluminiumoxid wird heiss filtriert. Nach dem Eindampfen des Filtrats erhält man 1,57 g (47 %) des Produktgemischs. Massenspektrum: 344, 346, 348 und 350 (M⁺: Basispeak).

### B) Herstellungsbeispiele:

Beispiel B1: 2,3,8,9-Tetrachlor-6,11-diphenyloxy-naphthacen-5,12-dion.
2,32 g (5 mmol) Verbindung gemäss Beispiel A1, 1,41 g (15 mmol) Phenol, 4,15 g (30 mmol) Kaliumcarbonat und 100 ml Tetrahydrofuran werden 5 Stunden am Rückfluss erhitzt. Das Gemisch wird unter Rühren auf Wasser gegossen und dann filtriert. Der Niederschlag wird zuerst mit Wasser und dann mit Methanol/Wasser gewaschen, getrocknet und dann aus o-Dichlorbenzol umkristallisiert. Man erhält die Titelverbindung in einer Ausbeute von 2,57 g (89 %) als gelbe Kristalle mit einem Smp. von 326-329 °C. Massenspektrum: 578/580/582 (M⁺: Basispeak).
Eine Lösung der Verbindung in Toluol zeigt bei Bestrahlung einen reversiblen Farbumschlag von gelb nach orange.

Beispiel B2: 2,3,6,8,9,11-Hexaphenyloxy-naphthacen-5,12-dion.
1,5 g (3,2 mmol) Verbindung A1, 2,43 g (25,8 mmol) Phenol, 4,45 g (32,3 g) Kaliumcarbonat und 120 ml N-Methylpyrrolidon werden 8 Stunden bei 150 °C gerührt. Das Gemisch wird in Tetrahydrofuran/Toluol/2N Salzsäure aufgenommen, die organische Phase abgetrennt, mit Wasser gewaschen, über Natriumsulfat getrocknet und dann eingedampft. Das Rohprodukt wird in Toluol gelöst und über Kieselgel chromatographiert. Man erhält die Titelverbindung in einer Ausbeute von 0,65 g (25 %) als gelb-orange Kristalle. Massenspektrum: 810 (M⁺: Basispeak).
Eine Lösung der Verbindung in Toluol zeigt bei Bestrahlung einen reversible Farbumschlag von gelb-orange nach rot.

Beispiel B3: Gemisch von 2,6,11- und 6,9,11-Triphenyloxy-naphthacen-5,12-dion.
0,5 g (1,45 mmol) Verbindung A2, 0,8 g (5,8 mmol) Kaliumcarbonat, 0,48 g (5,07 mmol) Phenol und 5 ml Dimethylsulfoxid werden 4 Stunden bei 100 °C gerührt. Das Reaktionsgemisch wird auf verdünnte Salzsäure gegossen und mit Tetrahydrofuran/Toluol (1:1) extrahiert. Die Extrakte werden mit gesättigter wässriger NaCl und Wasser gewaschen, über Natriumsulfat getrocknet und eingedampft. Man kristallisiert aus Toluol um und erhält 0,58 g (75 %) der Titelverbindung, Smp.: 220 bis 223 °C. Massenspektrum: 534 (M⁺: Basispeak).
Eine Lösung der Verbindung in Toluol zeigt bei Bestrahlung einen reversible Farbumschlag von gelb nach orange.

### C) Anwendungsbeispiele

Beispiel C1: 2,5 g Polystyrol werden unter Argon bei 60 °C in 15 ml Toluol gelöst. Nach 20 Minuten werden unter Lichtausschluss 25 mg der Verbindung gemäss Beispiel B1 zugegeben und 20 Minuten gerührt. Die Lösung wird mit einer 200 »m-Rakel bei 80 °C auf eine Glasplatte aufgetragen und anschliessend 60 Minuten bei 80 °C getrocknet. Man erhält einen freistehenden Film von etwa 30 »m Dicke. Der Film wird auf einer Quarzplatte im Probenraum eines Spektralphotometers montiert und über eine Quarzfaser und einen UV-Filter mit einer Xenonlampe (300 W) bestrahlt. In Abständen von 60 Sekunden wird die Bestrahlung unterbrochen und das Absorptionsspektrum gemessen. Das Spektrum ändert sich von gelb (optische Dichte 1 bei etwa 320 nm, 0,4 bei etwa 400 nm und 0 oberhalb 450 nm) nach rot (Absorptionsbande im Bereich von etwa 400-450 nm (maximale optische Dichte 0,65 bei 480 nm). Die Zeitkonstante der Umwandlung beträgt 140 Sekunden.
Für die Rückreaktion wird der UV-Filter durch ein gelbes Kantenfilter (Schott GG455) ausgetauscht. Die integrale Bestrahlungsstärke im Bereich von 430-540 beträgt 3 mW/ cm². Durch die Bestrahlung verschwindet die Absorptionsbande bei 400-450 nm bis auf eine optische Dichte von maximal 0,2 bei 480 nm. Die Zeitkonstante der Rückreaktion beträgt 110 Sekunden. Bei weiteren Bestrahlungszyklen bleiben diese Grenzwerte der optischen Dichte konstant.

Beispiel C2: Es wird analog Beispiel C1 verfahren, aber die Verbindung gemäss Beispiel B2 verwendet. Das Spektrum ändert sich von gelb (optische Dichte 1 bei etwa 340 nm, 0,35 bei etwa 400 nm und 0 oberhalb 450 nm) nach rot (maximale Absorption 0,6 bei 480-510 nm) mit einer Zeitkonstante von 180 Sekunden. Die Rückrektion (Bestrahlungsstärke 2,5 mW/cm²) erfolgt mit einer Zeitkonstante von 160 Sekunden (maximale optische Dichte von 0.15 bei 480 nm).

## Patentansprüche

1. Verbindungen der Formel I oder Mischungen solcher Verbindungen, 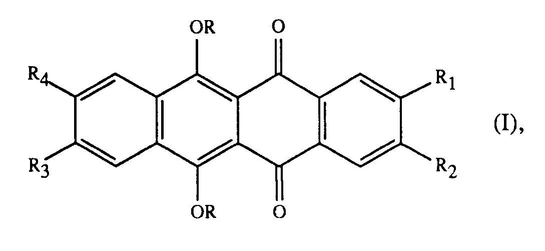 worin
R unsubstituiertes oder mit C₁-C₁₂-Alkyl, C₁-C₁₂-Alkoxy, C₁-C₁₂-Alkylthio, Phenyl, Benzyl, -CN, -CF₃, Halogen oder -COOR₅ substituiertes C₆-C₁₄-Aryl bedeutet und R₅ für H, C₁-C₁₈-Alkyl, Cyclohexyl, Cyclopentyl, Phenyl, C₁-C₁₂-Alkylphenyl, Benzyl oder C₁-C₁₂-Alkylbenzyl steht, und
mindestens einer der Reste R₁ bis R₄ -F, -Cl oder -Br darstellt oder unabhängig die Gruppe RO- bedeutet, und die anderen Reste von R₁ bis R₄ für -H, -F, -Cl oder -Br stehen.

2. Verbindungen gemäss Anspruch 1, worin R in Formel I unsubstituiertes oder substituiertes C₆-C₁₀-Aryl ist.

3. Verbindungen gemäss Anspruch 2, worin R unsubstituiertes oder substituiertes Phenyl, 1- oder 2-Naphthyl bedeutet.

4. Verbindungen gemäss Anspruch 1, worin R in Formel I unsubstituiert oder mit C₁-C₄-Alkyl, C₁-C₄-Alkoxy, C₁-C₄-Alkylthio, -F, -Cl, -Br oder -COOR₅ substituiert ist, wobei R₅ H oder C₁-C₁₈-Alkyl bedeutet.

5. Verbindungen gemäss Anspruch 1, worin in Formel I mindestens einer der Reste R₁ bis R₄ eine Gruppe RO- oder -F, -Cl oder -Br darstellt, und die anderen Reste von R₁ bis R₄ für H stehen.

6. Verbindungen gemäss Anspruch 1, worin R₁ oder R₄, oder R₁ und R₃ oder R₄, oder R₁ und R₂ oder R₁ bis R₄ eine Gruppe RO-, -F, -Cl oder -Br darstellen.

7. Verbindungen gemäss Anspruch 1, worin R₁ oder R₄, oder R₁ und R₃ oder R₄, oder R₁ und R₂ oder R₁ bis R₄ eine Gruppe RO- oder -Cl darstellen.

8. Verbindungen gemäss Anspruch 1, worin R unsubstituiertes Phenyl darstellt.

9. Verbindungen gemäss Anspruch 1, worin die Verbindungen der Formel I 6,11-Diphenyloxy-2-chlor-naphthacen-5,12-dion, 2,6,11-Triphenyloxy-naphthacen-5,12-dion, 6,11-Diphenyloxy-2,3,8,9-tetrachlor-naphthacen-5,12-dion und 2,3,6,8,9,11-Hexaphenyloxy-naphthacen-5,12-dion sind.

10. Verfahren zur Herstellung von Verbindungen der Formel I gemäss Anspruch 1, bei dem man 1 Mol einer Verbindung der Formel II 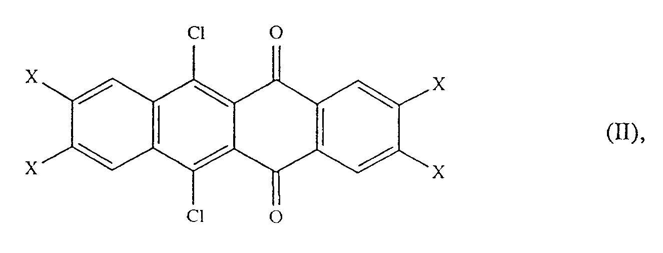 worin mindestens einer der Reste X für -F, -Cl oder -Br steht und die anderen Reste X für -H, -F, -Cl oder -Br stehen, in Gegenwart eines polaren aprotischen Lösungsmittels und bei erhöhter Temperatur mit mindestens 2 Mol einer Verbindung der Formel RO^{⊖}M^{⊕} umsetzt, worin R die in Anspruch 1 angegebenen Bedeutungen hat und M für ein Alkalimetall oder tertiäres Ammonium mit 3 bis 18 C-Atomen steht.

11. Verbindungen der Formel V 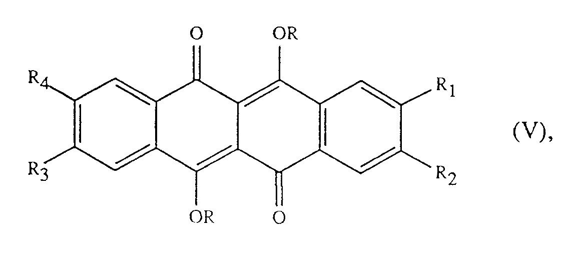 worin R, R₁, R₂, R₃ und R₄ die in Anspruch 1 angegebenen Bedeutungen haben, oder Mischungen solcher Verbindungen.

12. Zusammensetzung, enthaltend
a) ein farbloses organisches Lösungsmittel, ein Polymer oder ein organisches Glas oder ein Verbundglas, und
b) gelöst, eingemischt oder als Schicht auf mindestens einer Oberfläche eine Verbindung der Formel I oder V gemäss den Ansprüchen 1 oder 11 oder Mischungen davon.

13. Verwendung von Verbindungen der Formeln I oder V gemäss den Ansprüchen 1 oder 11 oder Mischungen davon als reversible photochrome Systeme zur Kontrastbildung oder Lichtabsorption.

14. Verwendung von Verbindungen der Formeln I oder V gemäss den Ansprüchen 1 oder 11 oder Mischungen davon zur reversiblen optischen Speicherung von Informationen.

## Claims

1. A compound of formula I or a mixture of such compounds, 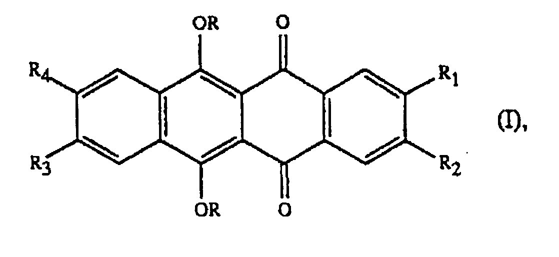 wherein
R is unsubstituted C₆-C₁₄aryl or C₆-C₁₄aryl which is substituted by C₁-C₁₂alkyl, C₁-C₁₂alkoxy, C₁-C₁₂alkylthio, phenyl, benzyl, -CN, -CF₃, halogen or -COOR₅, and R₅ is H, C₁-C₁₈alkyl, cyclohexyl, cyclopentyl, phenyl, C₁-C₁₂alkylphenyl, benzyl or C₁-C₁₂alkylbenzyl, and at least one of the radicals R₁ to R₄ is -F, -Cl or -Br, or is independently the group RO-, and the other radicals R₁ to R₄ are -H, -F, -Cl or -Br.

2. A compound according to claim 1, wherein R in formula I is unsubstituted or substituted C₆-C₁₀aryl.

3. A compound according to claim 2, wherein R is unsubstituted or substituted phenyl or 1- or 2-naphthyl.

4. A compound according to claim 1, wherein R in formula I is unsubstituted or substituted by C₁-C₄alkyl, C₁-C₄alkoxy, C₁-C₄alkylthio, -F, -Cl, -Br or -COOR₅, where R₅ is H or C₁-C₁₈alkyl.

5. A compound according to claim 1, wherein in formula I at least one of the radicals R₁ to R₄ is a group RO- or -F, -Cl or -Br, and the other radicals R₁ to R₄ are H.

6. A compound according to claim 1, wherein R₁ or R₄, or R₁ and R₃ or R₄, or R₁ and R₂ or R₁ to R₄ are a group RO-, -F, -Cl or -Br.

7. A compound according to claim 1, wherein R₁ or R₄, or R₁ and R₃ or R₄, or R₁ and R₂ or R₁ to R₄ are a group RO- or -Cl.

8. A compound according to claim 1, wherein R is unsubstituted phenyl.

9. A compound according to claim 1, wherein the compound of formula I is 6,11-diphenyloxy-2-chloronaphthacene-5,12-dione, 2,6,11-triphenyloxynaphthacene-5,12-dione, 6,11-diphenyloxy-2,3,8,9-tetrachloronaphthacene-5,12-dione or 2,3,6,8,9,11-hexaphenyloxynaphthacene-5,12-dione.

10. A process for the preparation of a compound of formula I according to claim 1, which comprises reacting 1 mol of a compound of formula II 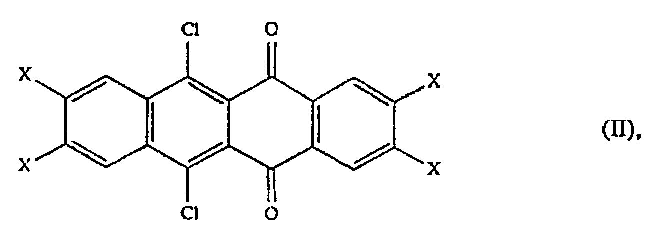 wherein at least one of the radicals X is -F, -Cl or -Br, and the other radicals X are -H, -F, -Cl or -Br, in the presence of a polar aprotic solvent and at elevated temperature, with at least 2 mol of a compound of formula RO^{⊖}M^{⊕}, wherein R is as defined in claim 1 and M is an alkali metal or tertiary ammonium containing 3 to 18 carbon atoms.

11. A compound of formula V 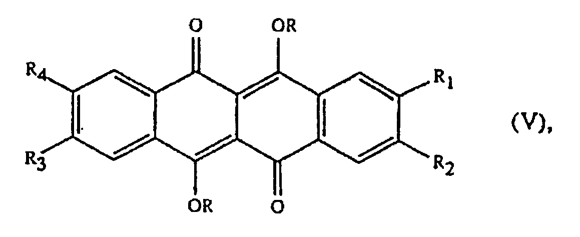 wherein R,R₁,R₂,R₃ and R₄ are as defined in claim 1, or a mixture of such compounds.

12. A composition comprising
a) a colourless organic solvent, a polymer or an organic glass or a compound glass, and
b) dissolved, incorporated therein or present as a layer on at least one surface, a compound of formula I or V according to either claim 1 or claim 11 or a mixture thereof.

13. Use of a compound of formula I or V according to either claim 1 or claim 11, or a mixture thereof, as reversible photochromic system for contrast formation or light absorption.

14. Use of a compound of formula I or V according to either claim 1 or claim 11, or a mixture thereof, for the reversible optical storage of information.

## Revendications

1. Composés de formule I ou mélanges de tels composés : 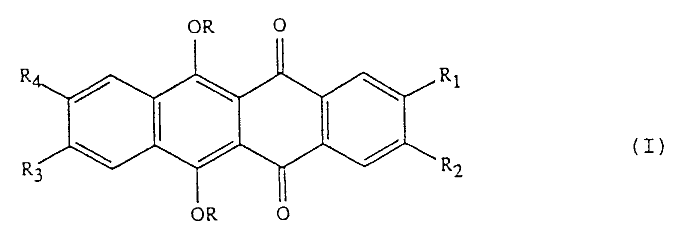 où
R représente un aryle en C₆-C₁₄ non substitué ou substitué par alkyle en C₁-C₁₂, alcoxy en C₁-C₁₂, alkylthio en C₁-C₁₂, phényle, benzyle, -CN, -CF₃, halogène ou -COOR₅ et R₅ représente H, un alkyle en C₁-C₁₈, un cyclohexyle, un cyclopentyle, un phényle, un alkylphényle en C₁-C₁₂, un benzyle ou un alkylbenzyle en C₁-C₁₂, et
au moins un des radicaux R₁ à R₄ représente -F, -Cl ou -Br ou, indépendamment, le groupe RO- et les autres radicaux de R₁ à R₄ représentent -H, -F, -Cl ou -Br.

2. Composés selon la revendication 1, où R dans la formule I est un aryle en C₆-C₁₀ non substitué ou substitué.

3. Composés selon la revendication 2, où R signifie un 1- ou 2-naphtyle, un phényle non substitué ou substitué.

4. Composés selon la revendication 1, où R dans la formule I est non substitué ou substitué par alkyle en C₁-C₄, alcoxy en C₁-C₄, alkylthio en C₁-C₄, -F, -Cl, -Br ou -COOR₅, où R₅ représente H ou un alkyle en C₁-C₁₈.

5. Composés selon la revendication 1, où dans la formule I, au moins un des radicaux R₁ à R₄ représente un groupe RO- ou -F, -Cl ou -Br, et les autres radicaux de R₁ à R₄ représentent H.

6. Composés selon la revendication 1, où R₁ ou R₄, ou R₁ et R₃ ou R₄, ou R₁ et R₂, ou R₁ à R₄ représentent un groupe RO-, -F, -Cl ou -Br.

7. Composés selon la revendication 1, où R₁ ou R₄, ou R₁ et R₃ ou R₄, ou R₁ et R₂, ou R₁ à R₄ représentent un groupe RO- ou -Cl.

8. Composés selon la revendication 1, où R représente un phényle non substitué.

9. Composés selon la revendication 1, où les composés de formule I sont la 6,11-diphényloxy-2-chloro-naphtacène-5,12-dione, la 2,6,11-triphényloxy-naphtacène-5,12-dione, la 6,11-diphényloxy-2,3,8,9-tétrachloro-naphtacène-5,12-dione et la 2,3,6,8,9,11-hexaphényloxy-naphtacène-5,12-dione.

10. Procédé pour la préparation de composés de formule I, selon la revendication 1, en faisant réagir 1 mole d'un composé de formule II : 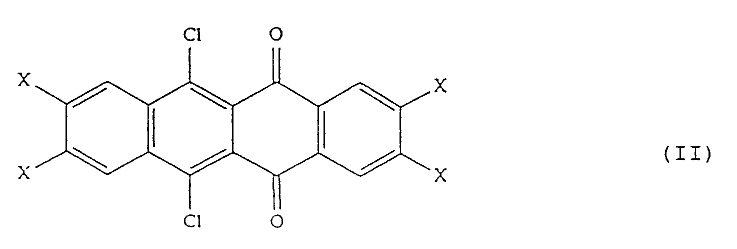 dans laquelle au moins un des radicaux X représente -F, -Cl ou -Br et les autres radicaux X représentent -H, -F, -Cl ou -Br, en présence d'un solvant aprotique polaire et à température élevée, avec au moins 2 moles d'un composé de formule RO^{⊖}M^{⊕}, où R a les significations données dans la revendication 1, et M représente un métal alcalin ou un ammonium tertiaire avec 3 à 18 atomes de carbone.

11. Composés de formule V : 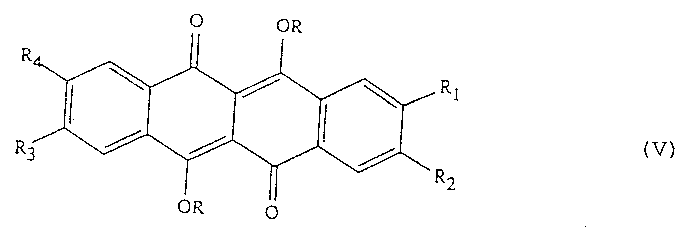 où R, R₁, R₂, R₃ et R₄ ont les significations données dans la revendication 1, ou les mélanges de ces composés.

12. Composition contenant :
a) un solvant organique incolore, un polymère ou un verre organique ou un verre feuilleté, et
b) un composé de formules I ou V selon la revendication 1 ou 11 dissous, en mélange ou sous forme de couche sur au moins une surface, ou leurs mélanges.

13. Utilisation des composés de formules I ou V selon les revendications 1 ou 11 ou de leurs mélanges en tant que systèmes photochromes réversibles pour la formation de contraste ou pour l'absorption de la lumière.

14. Utilisation des composés de formules I à V selon les revendications 1 ou 11 ou de leurs mélanges pour la mémorisation optique réversible des informations.
